# EUROPEAN PATENT SPECIFICATION

(11) **EP 1 998 734 B1**
(45) Date of publication and mention of the grant of the patent: **27.11.2013**
(21) Application number: 06828474.4
(22) Date of filing: 14.12.2006
(51) Int. Cl.: A61K 6/00, A61K 8/24, A61Q 11/00

(54) **AN AGENT FOR THE PREVENTION AND TREATMENT OF THE INITIAL DENTAL CARIES**
MITTEL ZUR PROPHYLAXE UND THERAPIE VON ANFÄNGLICHER ZAHNKARIES
AGENT DE PREVENTION ET DE TRAITEMENT DES CARIES DENTAIRES INITIALES

(30) Priority: 19.01.2006 CZ 20060040
(43) Date of publication of application: 10.12.2008
(73) Proprietor: Ceská hlava s.r.o., Svatý Jan pod Skalou (CZ)
(72) Inventor: MARYSKA, Martin, 100 00 Praha 10 (CZ)
(74) Representative: Plicka, Josef
(86) International application number: PCT/CZ2006/000091
(87) International publication number: WO 2007/082496

(56) References cited:
- WO-A-98/07448
- WO-A-99/20237
- CN-A- 1 723 915
- RU-A- 2004 106 053
- DATABASE EPODOC EUROPEAN PATENT OFFICE, THE HAGUE, NL; XP002460779 & BG 92 020 A (DJULGEROVA [BG]; ATANASOV [BG]; DIMITROVA [BG]) 31 January 1995 (1995-01-31)

## Description

### Field of the Invention

The invention deals with an agent for the prevention and treatment of the initial dental caries.

### Background of the Invention

The dental caries represents a chronic infectious disease that progresses very slowly in the majority of individual persons. The disease may affect the enamel, the dentine or the cement and it is not characterized by any selflimiting factors which means that it may lead to a complete destruction of the tooth if it is not treated.

The tooth caries occurring on the surface of the dental enamel is a process reflecting the metabolic activity taking place in the biofilm of the dental plaque. In the course of time the balance between the mineral surface of the enamel and the surrounding dental plaque liquid is disturbed. The loss of the hard tooth tissue by demineralization results eventually in the formation of a carious lesion.

The tooth caries is a multifactorial disease with a variety of causes. Some factors are necessary for the development of the caries, in particular the presence of the dental plaque biofilm on the enamel surface, the presence of carbohydrates and the susceptible surface of the enamel; other factors are supplementary, eg the composition of the food and the frequency of its consumption, fluorides, buffer capacity of the saliva, etc.

The development and the progression of the initial decay lesion are described by some experimental studies.

The methods preventing the formation of the dental caries have made a great progress in the last half a century. These methods encompass the regular mechanical removal of the dental plaque biofilm by various techniques and antimicrobial agents, the dietary counselling aimed at decreasing the frequency of consumption of sweet food and drinks as well as the most successful preventive techniques based on the application fluoride ions into the enamel hydroxyapatite. The prevention of the dental caries by fluorides set out from the idea of the substitution of the hydroxyl groups in the enamel hydroxyapatite by fluorine ions leading to its resistance to the dissolution in acids; the formed fluorohydroxyapatite or fluorapatite makes the enamel surface extraordinarily resistant against the dental caries development.

In addition to this embedment of fluoride ions in the enamel hydroxyapatite through the systemic supply of fluorides (fluoridated drinking water, NaF tablets, fluoridated salt and fluoridated milk) an extraordinarily efficient local action of fluoride ions on the surface layer of the enamel was discovered in the 1980s. It was found that if fluoride ions are present in the saliva or in the dental plaque liquid they enter spontaneously into the surface layer of the enamel into the depth of about 20 to 30 *µ*m thus making it extremely resistant to the acid attack. At present, this local action of fluorides is regarded as the basic method of dental caries prevention.

At the time present a wide range of various dental hygiene means characterized by local fluoride action are being used with the aim to prevent the dental caries: fluoridated tooth pastes, fluoride gels to be applied at the dental chair as well as in daily use at home, mouthrinses with a higher fluorine content for mouth washing, fluoridated dental flosses and fluoridated chewing gums. Various calcium phosphates that should encourage the formation of dental enamel hydroxyapatite also occur in a variety of toothpastes. However, the minimum size of all ingredients added so far into currently used toothpastes and other fluoride containing agents are in the order of several to tens of micrometers. Such huge particles are not able to penetrate into carious microdefects in the tooth enamel and influence their repair.

A pronounced development has taken place in the field of another extraordinary invention in the past decade - the application of the nanotechnologies. Nanomaterials with the particle size ranging from 10 to 100 nm exhibit other properties than identical materials composed of the grains having the size of hundreds of nanometers or being even larger. Among other things, also the processes suitable for the preparation of nanoparticles of hydroxyapatite and other well-defined calcium phosphates were developed. Their application in the dentistry is also mentioned in patent literature but no such material has been applied in the practice so far. Obviously, this is due to the fact that the techniques that would enable to investigate the tooth microstructure and its changes resulting from the use of therapeutic processes "in vivo" are not currently available. The electron microscopy is applied almost exclusively to the investigation into the enamel microstructure by using extracted teeth on which, however, various treatment effects can not be studied any longer.

In RU 2 280 432 a process for the preparation of a composition for the treatment of initial dental caries which comprises calcium and phosphate and fluoride ions is disclosed. The disclosed gel composition is intended for the fluoridation of dental enamel. Unlike the claimed composition, the resulting fluorine containing gel is based on carboxymethylcellulose as a gel-forming agent.

A mixture containing nanoparticles of hydroxyapatite used for a rapid repair of enamel defects is described in the technical literature (K. Yamagishi, K. Onuma, T. Suzuki, F. Okada, J. Tagami, M. Otsuki, P. Senawangse: A synthetic enamel for rapid tooth repair. Nature, vol. 433, 24 February 2005, p. 819). Nevertheless, the workers use a mixture of HAP nanoparticles in a very acid environment, which affects unfavorably the mucous membrane in the oral cavity.

The subject of the present invention is to offer an agent that - in comparison with the known materials used up to now - would enable an even more efficient prevention and cure of the initial dental caries lesion.

### Summary of Invention

The above mentioned subject has been achieved by using the agent for prevention and curing of the initial tooth caries according to this invention whose substance consists in the fact that it is composed of a phosphate-calcium gel with the Ca/P mass ratio ranging from 1.2 to 2.2 and with the bond energy of the 2p electron of phosphorus equal to 131 eV to 136 eV and, optionally, of at least one auxilliary dental substance, too. To a good advantage, the agent according to the invention can further contain the aqueous solution of methyl cellulose or/and chlorohexidine gluconate. Proteins of the enamel matrix (amelogenine, ameloblastine, enameline and tufteline), monetite, sodium fluoride and taste improving additives may also be used as additional additives.

This agent can be applied in form of toothpaste, paste, chewing gum, coated dental floss, foam, gel or varnish.

### Description of the pictures shown in Figures

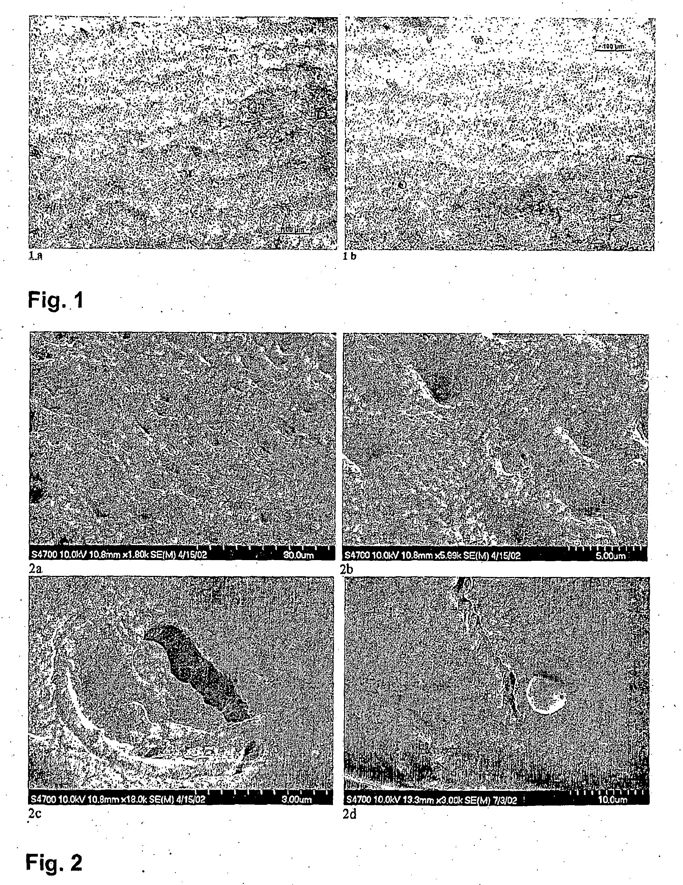

On the attached drawings:
The surface make-up of the enamel of a 14-year-old person with symptoms of an initial decay process (the porosity starting to develop) are shown in Fig. 1a and Fig. 1b. Considered from the viewpoint of current present-day diagnostic methods this would be a tooth without any sign of the clinically identifiable decay activity. Perikymata are visible as well as the contours of the enamel prisms. The pictures were taken "in vivo" with the aid of an optical microscope.
Fig. 2 This Figure shows the development of a sub-surface porosity: Fig. 2a - individual pores at the enamel surface; Fig. 2b, c, d - the pore coalescence (various types). All pictures were obtained "in vitro" with the aid of a scanning electron microscope (SEM).

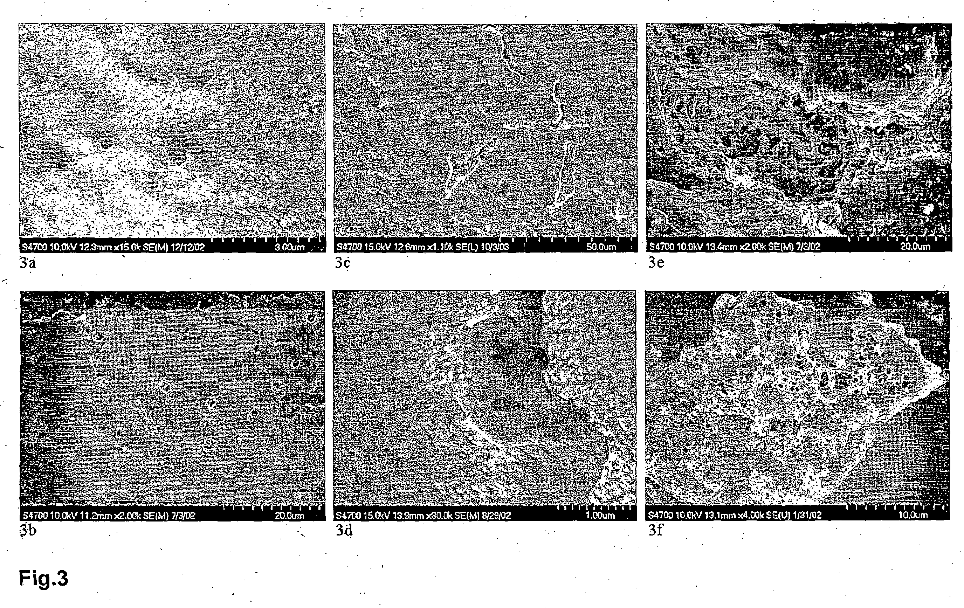

Fig. 3 Figures 3a through 3f show, in detail, individual stages of the development in the initial decay lesion. Fig. 3a shows the formation of pores and their coalescence, Fig. 3b shows the porosity development, the pore coalescence and micro-fissures, Fig. 3c shows the pore coalescence and micro-cavitation, Fig. 3d shows pores in the wall of a micro-fissure, Fig. 3e shows the micro-cavitation and the beginning pitting effect in the depth of a micro-cavity and Fig. 3f shows the micro-cavitation of a initial decay with a broken-off surface layer of the enamel. All pictures were taken "in vitro" with the aid of a scanning electron microscope.

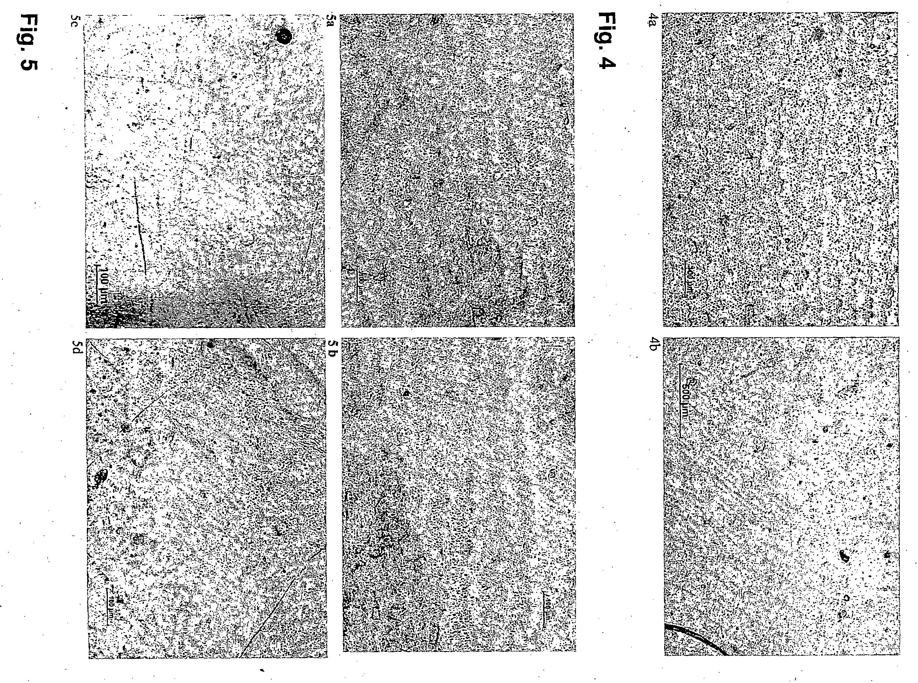

Fig. 4 Figures 4a and 4b show reparation of defects in the surface of tooth by calciumfosfate gel tratment. 4a picture shows original surface tooth in face of calciumfosfate gel application, picture 4b shows surface the same tooth after application of gel, which was aggradation on teeth by tooth-brush 1 month 1x every day - the microdefects were fill and was created continuous phosphate layer. Photos are of optical microscope effected in vivo.
Fig. 5 Fig. 5a through Fig. 5d demonstrates the reparative effect of a calcium phosphate gel "in vivo". Fig. 5a and 5b show the initial surface state of the first upper premolar (considered from the viewpoint of current present-day diagnostic methods this would be a tooth without any sign of the clinically identifiable decay activity - see Fig. 1a and 1b), Fig. 5c and 5d demonstrate a reparative action of a calcium phosphate gel applied in the form of a toothpaste for 14 days; two-minute tooth cleaning twice a day, 14-year-old patient. The pictures made "in vivo" with the aid of an optical microscope.

### Examples

### Preparation of the gel with the Ca/P mass ratio = 2.1562

### 1. Preparation of the Ca(NO₃)₂ .4H₂O solution

116.8 g of Ca(NO₃)₂ . 4H₂O are dissolved at 37 °C in 50 milliliters of the mixture of distilled water with ethanol at a ratio 1:1 and the pH value of the solution obtained in such a way is adjusted with concentrated ammonia to pH = 9 (approximately 0.4 milliliters).

### 2. Preparation of the (NH₄)₂HPO₄ solution

39.1 g of (NH₄)₂HPO₄ are dissolved in 50 milliliters of distilled water at 37 °C and the pH value of the solution prepared in such a way is adjusted with concentrated ammonia to pH = 9 (4 milliliters).

Both solutions are mixed together at a temperature of 37 C, the mixture is stirred continuously and intensely with a magnetic stirrer while the solution of calcium nitrate is poured into the solution of ammonium phosphate. The final product is filtered several times immediately after the end of the stirring lasting for 3 minutes. The product on the filter is first rinsed once with warm distilled water, then with the water/ethanol solution (volume ratio 1:1) and, finally, with ethanol only. The filtering is carried out with the aid of a water jet pump by using the S4 frit and "blue ribbon" paper filter.

The resulting product is characterized by the bond energy of the P₂ₚ phosphorus electron ranging from 135 eV to 132 eV and by the Ca/P mass ratio identical with that in hydroxyapatite, ie 2.16.

### Example 1

5 g of the product prepared in the above mentioned way is dispersed in the 2-percent solution of tylose and 25 milliliters of 0.1-percent chlorohexidine are added to the mixture obtained; this mixture is then homogenized by ultrasound (100 W for a period lasting 5 minutes). The final product is thin and shows a tendency towards sedimentation; therefore, it is suitable for immediate application. It may be necessary to stir it thoroughly before use.

### Example 2

5 g of the product are dispersed in the 2-percent solution of tylose (tylose dissolved in 50 milliliters of water, 25 milliliters of the 0.1-percent chlorohexidine added to the solution and made up to the mark of 100 milliliters). First, a small amount of this tylose solution is added to the weighed amount of gel (5 g to obtain a mixture containing 5 %), the mixture is stirred thoroughly, then the remaining quantity of tylose is added, the mixture is stirred thoroughly once again with a glass rod and, subsequently, the homogenization by ultrasound similar to that mentioned in Example 1 takes place. The resulting product is more viscous than that mentioned in Example 1 but it still exhibits a tendency towards sedimentation and, therefore, it is also suitable for immediate use or it may be necessary to stir it thoroughly before use.

### Example 3

10 g of the product are dispersed in the 4-percent solution of tylose (tylose dissolved in 60 milliliters of water, 25 milliliters of the 0.1-percent chlorohexidine added to the solution and made up to the mark of 100 milliliters directly in the beaker). First, a small amount of this solution of tylose is added to the weighed amount of gel (5 g to obtain a mixture containing 5 %), the mixture is stirred thoroughly, then the remaining quantity of tylose is added, the mixture is stirred thoroughly once again with a glass rod and, subsequently, the homogenization by ultrasound similar to that mentioned in Example 1 takes place. The resulting consistency of the product meets the requirements for current applications, ie it sediments negligibly.

### Example 4

10 g of the product are dispersed in the 4-percent solution of tylose (tylose dissolved in 60 milliliters of water, 25 milliliters of the 0.1-percent chlorohexidine added to the solution and made up to the mark of 100 milliliters directly in the beaker). First, a small amount of this solution of tylose is added to the weighed amount of gel (5 g to obtain a mixture containing 5 %), the mixture is stirred thoroughly, then the remaining quantity of tylose and 5 milliliters of a strawberry syrup are added, the mixture is stirred thoroughly once again with a glass rod and, subsequently, a five-minute treatment by ultrasound takes place. The resulting consistency of the product is the same as that in Example 3.

### Example 5

The same procedure as that described in Example 4 is used except for the fact that 1 percent of a mixture of proteins of the glassy matrix (amelogenine, ameloblastine, enameline and tufteline) is added.

### Industrial applicability

The agent as described in this invention can be used practically in the form of rinsing mouth suspension, rinsing suspension from artificial saliva, gel compress containing a gel with the Ca/P mass ratio equal to 1.2 ... 2.2, toothpaste containing sol, respectively gel with the Ca/P mass ratio equal to 1.2 ... 2.2, toothpastes to be used in conjunction with a standard or ultrasonic toothbrush, chewing gums containing a gel with the Ca/P mass ratio equal to 1.2 ... 2.2, high-viscosity mixtures containing a gel with the Ca/P mass ratio equal to 1.2 ... 2.2 to be used a) as a fissure sealing agent, b) on the interdental toothbrush to treat the approximal surfaces of the tooth, c) on dental thread to treat approximal surfaces.

The agent according to invention is suitable for both the preventative care and curative purposes aimed at treating the initial tooth caries.

As regards the home care, toothpastes, pastes, chewing gums, dental threads and foam come into consideration. Gel, powder and paint could then be used within the framework of the professional care.

## Claims

1. The agent for the prevention and treatment of the initial dental caries, **characterized in that** it is formed by a phosphate-calcium gel with the Ca/P mass ratio ranging from 1.2 to 2.2 and with the bond energy of the 2p phosphorus electron of 131 eV to 136 eV and, optionally, containing at least one auxiliary dental substance.

2. The agent according to Claim 1, **characterized in that** it further contains an aqueous solution of methyl cellulose or/and chlorohexidine gluconate.

## Patentansprüche

1. Agens für Verhütung und Behandlung der Anfangszahnkaries, **dadurch gekennzeichnet, dass** es aus Kalziumphosphatgel mit Gewichtsverhältnis Ca/P im Bereich von 1,2 bis 2,2 besteht und über die Bindungsenergie von 2p des Phosphorelektrons 131 eV bis 136 eV verfügt und gegebenfalls mindestens eine Dentalhilfssubstanz enthält.

2. Agens nach Anspruch 1, **dadurch gekennzeichnet, dass** es noch eine Wasserlösung der Methylcellulose oder/und des Chlorhexidinglukonats enthält.

## Revendications

1. Agent pour prévention et traitement de la carie dentale initiale, **caractérisé en ce qu'il** est formé par phosphatcalcium gélatineux avec rapport de masse Ca/P en limite de 1,2 a 2,2 et par l'énergie de liaison de 2p d'électron de phosphore 131 eV a 136 eV, le cas échéant l'agent comprend au moins une matiere dentale auxiliaire.

2. Agent selon la revendication 1, **caractérisé en ce qu'il** contient une solution aqueuse de méthylcellulose ou/et de chlorhexidingluconate.
